# EUROPEAN PATENT APPLICATION

(11) **EP 4 123 664 A1**
(43) Date of publication of application: **25.01.2023**
(21) Application number: 21187163.7
(22) Date of filing: 22.07.2021
(51) Int. Cl.: G16H 50/50

(54) **MODELLING THE HEART OF A SUBJECT**

(71) Applicant: Koninklijke Philips N.V., 5656 AG Eindhoven (NL)
(72) Inventor: WEESE, Rolf Jürgen, 5656 AE Eindhoven (NL); WAECHTER-STEHLE, Irina, 5656 AE Eindhoven (NL); GROTH, Alexandra, 5656 AE Eindhoven (NL); CLUITMANS, Matthijs Joseph Maria, 5656 AE Eindhoven (NL)
(74) Representative: Philips Intellectual Property & Standards

(57) **Abstract**

A mechanism for generating information usable for identifying a risk of arrhythmia. A plurality of heart models are constructed from medical imaging data of a subject's heart, each heart model being defined by a set of different values for one or more modifiable properties. The modifiable properties are those used in the generation of the heart model or in the definition of the heart model, and influence or affect simulation results using the heart model. Each heart model undergoes a plurality of simulations, each simulation being a simulation of a response of the heart model to a (simulated) stimulation of electricity at a different pacing location of the heart. Output data is generated containing indicators of all simulation results.

## Description

### FIELD OF THE INVENTION

The present invention relates to the field of cardiac monitoring and/or assessment, and in particular to the field of computer-implemented modelling of a heart.

### BACKGROUND OF THE INVENTION

There has been a growing interest in cardiac monitoring to make use of computer-implemented models of the heart (a "heart model") to assess or predict adverse cardiac activity, e.g. arrhythmia such as ventricular tachycardia. It is possible to construct a heart model from non-invasively acquired information about a subject, such as medical images and/or physiological data of the subject. This allows predictions about the cardiac health of the subject to be made without the need for an invasive procedure such as exploratory surgery. This information can be used, for instance, to guide a later surgical procedure such as ablation or placement of an interventional device (e.g. a pacemaker, a stent and so on).

Constructed heart models can also be used to simulate the effect of different cardiac conditions or triggers. This could allow simulated tests, which might otherwise be subject to medical or ethical concerns, to be performed, for improved analysis, assessment and diagnosis of the modelled heart.

One area in which heart models find use is in electrophysiologic simulations, which have been carried out for assessing atrial fibrillation and ventricular tachycardia. Typically, during such simulations a virtual heart, i.e. a heart model, is subjected to pacing from multiple locations to elicit reentrant arrhythmias. The reaction of the heart model can be used to assess or diagnose a condition of the heart.

N. A. Trayanova, F. Pashakhanloo, K. C. Wu & H. R. Halperin; Imaging-based simulations for predicting sudden death and guiding ventricular tachycardia ablation, Circulation: Arrhythmia and Electrophysiology. 2017 provides one example of using heart models to simulate reentrantreentrant arrhythmia and guide an ablation procedure.

### SUMMARY OF THE INVENTION

The invention is defined by the claims.

According to examples in accordance with an aspect of the current disclosure, there is provided a computer-implemented method of modelling the heart of a subject.

The computer-implemented method comprises: obtaining medical image data of the heart of a subject; constructing a plurality of different heart models of the heart of the subject by processing the medical image data, wherein each heart model is defined by a different set of one or more values for one or more modifiable properties of the heart model; for each heart model, performing two or more simulations of electrophysiologic activity, wherein each simulation is carried out using a different pacing location with respect to the heart model, to produce a respective two or more simulation results for the heart model; and generating output data, the output data comprising indicators of all simulation results produced for all heart models.

The present disclosure proposes an approach for (computer-implemented) modelling of the heart of a subject to produce output data. The output data comprises indicators of simulation results produced for each of a plurality of pacing locations on each of a plurality of different heart models. In the context of the present disclosure, one heart model is different to another heart model if one or more values (of one or more modifiable properties of the heart model) differ from one another.

It is herein recognized that, despite significant development of heart models, it is computationally difficult to generate a completely accurate and reliable heart model that directly represents all characteristics of the subject's heart. This is because the characteristics of each individual's heart (e.g. tissue conductivity, fiber length, fiber structure, cell action potential dynamics, peak sodium current, L-type calcium current, potassium current, longitudinal and transversal conduction velocities and so on) will differ. It is difficult (if not impossible) to obtain such characteristics non-invasively. Accordingly, heart models are usually generated based on (universally accepted) average values and/or parameters, as well as more easily or non-invasively identifiable features of the particular subject's heart (e.g. its shape, derivable from medical image data).

By producing simulation results for multiple, different heart models of the subject's heart, a sensitivity of the heart model to change can be understood. In particular, if the same simulation results are shared by multiple heart models, a confidence that the shared simulation results are accurate is increased. Similarly, if different heart models produce different simulation results, this would decrease a clinician's confidence in the simulation results.

Thus, a clinician (or further processing system) can be provided with useful clinical information for assessing an accuracy and/or confidence of simulation results produced using heart modelling techniques. This can be used to improve a diagnosis or assessment of the subject by drawing attention to possibly erroneous or misleading simulation results, reducing a chance of misdiagnosis or false assessment based on inaccurate results, as well as drawing attention to areas that may require particular attention, e.g. during later surgical procedures.

The present disclosure proposes to provide information about the reaction of multiple different heart models to different pacing locations. As previously explained, this gives useful information about the sensitivity of the simulation results. In particular, it is recognized that increased reliability and/or certainty about simulation results will be achieved if different heart models provide a same simulation result.

Preferably, the same pacing locations are used to perform simulations for different heart models. This provides an improved sensitivity analysis.

A pacing location represents a location on the intracardiac surface. Different pacing locations effectively represent a simulation of arrhythmic activity. Different forms of arrhythmic activity may result from stimulation at a specific location. Thus, just because no arrhythmic activity is seen after stimulation at one location does not mean that no arrhythmic activity will be seen after stimulation at another location.

The method may further comprise a step of processing the output data to generate, for each pacing location, a risk indicator representing a risk of arrhythmic activity for each pacing location.

In some examples, the arrhythmic activity may be ventricular tachycardia, such that the risk indicator represents a risk of ventricular tachycardia resulting from a pacing at the pacing location.

The method may further comprise a step of processing the output data to generate an overall risk indicator representing a risk of arrhythmic activity for the subject.

In some examples, the differences between different sets of one or more values for one or more modifiable properties represent variations between different individuals, variations between different population cohorts, measurement uncertainty and/or the influence of different drugs and/or treatments on the heart.

The simulation result may be a measure of arrhythmic activity. In particular examples, the measure of arrhythmic activity is a measure of a predicted length of reentrant arrhythmia.

In at least one embodiment, each indicator comprises a classification of whether or not arrhythmia is predicted, the classification being produced by processing the measure of arrhythmic activity.

Each heart model may be an electrophysical model of cardiac activity, and may comprise an ionic model of cardiac activity. An electrophysical model of cardiac activity is any heart model that can simulate or model the effect of electrical activity in the heart, e.g. a response of the heart to an electrical impulse at a particular pacing location (e.g. to simulate reentrant arrhythmia).

The one or more modifiable properties may comprise one or more electrophysiological properties of tissue represented by each heart model. Electrophysical properties include any properties that affect an electrophysical modelling of the model, such as tissue conductivity, fiber length, fiber structure, cell action potential dynamics, peak sodium current, L-type calcium current, potassium current, longitudinal and transversal conduction velocities.

The one or more modifiable properties of the heart may comprise an amount and/or location of scar tissue, gray zone tissue and/or non-infarcted tissue of the heart.

Embodiments may further comprise a step of outputting the output data to a user interface or processor. Some embodiments comprise a step of controlling a user interface to provide a visual representation of the output data.

There is also proposed a computer program product comprising computer program code means which, when executed on a computing device having a processing system, cause the processing system to perform all of the steps of any herein described computer-implemented method.

There is also proposed a processing system configured to model the heart of a subject. The processing system comprises: an input interface configured to obtain medical image data of the heart of a subject; and a processing arrangement configured to: construct a plurality of different heart model of the heart of the subject by processing the medical image data, wherein each heart model is defined by a different set of one or more values for one or more modifiable properties of the heart model; for each heart model, perform two or more simulations of electrophysiologic activity, wherein each simulation is carried out using a different pacing location with respect to the heart model, to produce a respective two or more simulation results; and generate output data, the output data comprising indicators of all simulation results produced for all heart model, wherein the processing system optionally further comprises an output interface configured to output the output data.

The processing system and/or processing arrangement may be configured to perform any herein described method, just as any herein described method may be configured to carry out the steps performed by any herein described processing system and/or processing arrangement.

There is also proposed a system comprising: the processing system previously described; and a user interface, wherein the processing arrangement is configured to control the user interface to provide a visual representation of the output data.

There is also proposed a system comprising: the processing system previously described and a medical imaging system configured to generate the medical imaging data obtained by the processing system.

There is also proposed a system comprising: the processing system previously described and a memory configured to store and provide the medical imaging data obtained by the processing system.

Of course, embodiments may provide a system comprising the user interface, the medical imaging system and/or the memory.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a method according to an embodiment;
Figure 2 illustrates an approach for generating a heart model for use in one or more embodiments;
Figure 3 illustrates a method according to an embodiment;
Figure 4 illustrates a processing system according to an embodiment; and
Figure 5 illustrates a system according to an embodiment.

### DETAILED DESCRIPTION OF THE EMBODIMENTS

The invention will be described with reference to the Figures.

The detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The Figures are merely schematic and are not drawn to scale. It should also be understood that the same reference numerals are used throughout the Figures to indicate the same or similar parts.

The current disclosure provides a mechanism for generating information usable for identifying a risk of arrhythmia. A plurality of heart models are constructed from medical imaging data of a subject's heart, each heart model being defined by a set of different values for one or more modifiable properties. The modifiable properties are those used in the generation of the heart model or in the definition of the heart model, and influence or affect simulation results using the heart model. Each heart model undergoes a plurality of simulations, each simulation being a simulation of a response of the heart model to a (simulated) stimulation of electricity at a different pacing location of the heart. Output data is generated containing indicators of all simulation results.

Embodiments are based on the realization that generating multiple, different heart models of the subject allows for a more robust simulation testing strategy, as consistent results across different heart models is indicative that the consistent result will accurately represent the true characteristics of the subject's heart. Conversely, inconsistent results across different heart models indicates that the modelling of the subject's heart is less accurate. This provides useful information for assessing the condition of the subject, and reduces the likelihood that an inaccurate assessment or analysis of the subject's will take place (e.g. due to erroneous or inaccurate simulation of the heart).

Embodiments may be employed in any suitable clinical environment in which medical imaging data of the subject's heart is obtained, e.g. during assessment of the subject's cardiac activity.

In the context of the present disclosure, the term "obtain" medical image data is meant to include passive receipt of such data (e.g. as they are provided at the input by another device) and an active receipt of such data (e.g under control of the processor that sends out a command to another device to have it provide the data at the output. Such other device may be a data storage device such as memory or an interventional device such as a catheter or other.

In the context of the present disclosure, a "heart model" is a representation of the heart of a subject as a computer model. The heart model may, for instance, be an electrophysical model or representation of the heart. The heart model may represent the electrophysiological activities at an organ level.

Inl particular examples, the heart model may comprise one or more ionic models of cardiac activity. An ionic model of cardiac activity may represent electrophysiological properties at a cellular level.

Heart models are defined by superset of modifiable parameters/properties, which are used in the generation of the heart model and/or to define properties of the heart model itself. For instance, one set of modifiable parameters/properties may be used to define which tissue types of different areas of the heart (which controls electrophysical properties of said areas). Another set of modifiable parameters/properties may be used to define the values of electrophysical properties assigned to particular areas of the model. Conventionally, measured properties of the subject or global/cohort approximations or averages are used to define the values of the superset of modifiable parameters/properties.

Suitable heart models for use in the present disclosure are well known in the art. Examples of suitable heart models are described by:
N. A. Trayanova, F. Pashakhanloo, K. C. Wu & H. R. Halperin; Imaging-based simulations for predicting sudden death and guiding ventricular tachycardia ablation, Circulation: Arrhythmia and Electrophysiology. 2017;
N. Smith et al.; euHeart: personalized and integrated cardiac care using patient-specific cardiovascular modelling. Interface Focus 2011, 1(3), 349 - 364;
H. Arevalo et al.; Arrhythmia risk stratification of patients after myocardial infarction using personalized heart models; Nature Communications 2016, 7, 11437; and
C. Mendonca Costa, G. Plank, C. A. Rinaldi, S. A. Niederer & M. J. Bishop; Modeling the Electrophysiological Properties of the Infarct Border Zone, Frontiers in Physiology 2018, 9, 356.

Numerous other examples of suitable heart models can be found in the prior art, and would be readily apparent to the skilled person. The present invention proposes an approach that makes use of existing approaches for generating heart models and simulating arrhythmia using generated heart models.

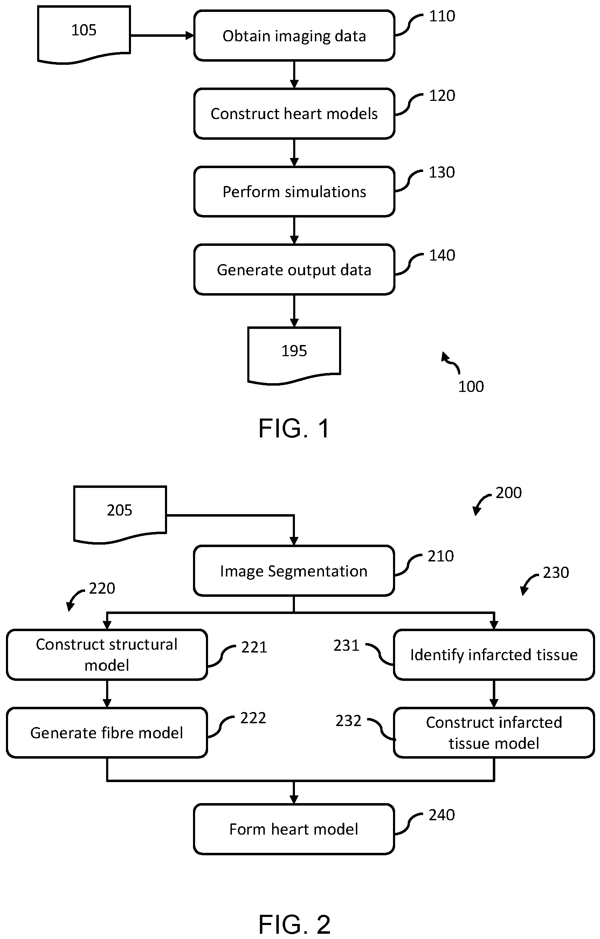

Figure 1 illustrates a method 100 according to an embodiment of the invention.

The method 100 is configured to generate output data 195 that contains indicators of a plurality of simulation result sets. Each set of simulation results represents the simulation results for a different heart model, with different simulations results within any particular set representing different pacing locations during a simulation using the heart model of that set.

The method 100 comprises a step 110 of obtaining medical image data 105. The medical image data is of a heart, i.e. contains data representing a heart, of a subject. The medical image data may be obtained from or provided by a medical imaging system and/or a memory system, e.g. which stores data originally generated by a medical imaging system. Any suitable form of medical imaging data may be used, for instance, X-ray or computed tomography (CT) imaging data, magnetic resonance (MR) imaging data, ultrasound imaging data, position emission tomography (PET) imaging data and so on.

The method 100 then processes the medical imaging data 105 in a step 120, which comprises constructing a plurality of different heart models of the heart of the subject by processing the medical image data. Each heart model is defined by a different set of one or more values for one or more modifiable properties or parameters of the heart model.

Thus, multiple versions or forms of heart model are generated in step 120, with each heart model being associated with or defined by a different set of parameter values. Put another way, each heart model may have different parameter settings that define, affect, influence or otherwise control the outcome of a simulation performed using the heart model.

Various modifiable properties or parameters of the heart model are envisaged for use in the present disclosure. Generally, the modifiable properties are those that will affect or influence the outcome of a simulation performed using the heart model. The modifiable properties comprise properties that are used in the generation of the heart model(s) and/or properties that define elements of the heart model (e.g. for use during later simulation).

By way of example, the one or more modifiable properties may comprise an amount and/or position of scar tissue, gray-zone tissue and/or non-infarcted tissue in the heart. In some examples, the one or more modifiable properties comprise one or more electrophysical properties of tissue making up the heart. Examples of such properties include tissue conductivity, fiber length, fiber structure, cell action potential dynamics, peak sodium current, L-type calcium current, potassium current, longitudinal and transversal conduction velocities and so on. In some example, the one or more modifiable properties may comprise information on a (potential) treatment or drug/medication that would affect the operation of the heart.

More detailed examples of such properties are provided later in this disclosure.

The method 100 then performs a step 130 of, for each heart model, performing two or more simulations of electrophysiologic activity, wherein each simulation is carried out using a different pacing location with respect to the heart model, to produce a respective two or more simulation results for the heart model. Each simulation produces a simulation result.

In performing the simulation of a heart model for a particular pacing location, an electrical impulse is simulated at the pacing location on the heart model. The response of the heart model is then monitored in order to generate the simulation result of the simulation. This process effectively performs ventricular tachycardia testing, i.e. to test whether there is correct contraction of the heart for a particular pacing location, and thereby simulates a potential cardiac event of the heart. In other words, simulating an electrical impulse or electrical signal at a particular pacing location simulates the effect or risk of ventricular tachycardia for the heart.

The simulation result may, for instance, comprise a predicted length of time for which re-entrant arrhythmia persists in the heart model following electrical stimulation at the pacing location. The simulation result may, for instance, comprise a predicted severity of re-entrant arrhythmia (e.g. how much the re-entrant arrhythmia affects the operation of the heart). Other suitable examples of simulation results will be apparent to the skilled person, and may depend upon the precise cardiac event under investigation (i.e. be embodiment-dependent).

Various and more detailed approaches for performing simulations of a heart model, using different pacing locations, will be apparent to the skilled person. H. Arevalo et al.; Arrhythmia risk stratification of patients after myocardial infarction using personalized heart models; Nature Communications 2016, 7, 11437 provides one example approach for performing appropriate simulations. S. Rapaka et al.; LBM-EP: Lattice-Boltzmann Method for Fast Cardiac Electrophysiology Simulation from 3D Images. MICCAI 2012, Part II, LNCS 7511, 33 - 40 provides another example approach, which benefits from being less computational expensive to perform, and can therefore be used to reduce computation time.

Step 130 may comprise performing two or more simulations (e.g. for different heart models and/or pacing locations) simultaneously, i.e. in parallel. As simulation can be a resource-intensive activity, this embodiment would significantly increase the speed of performing step 130.

The same or different pacing locations may be used for different heart models. Where the same pacing locations are used, it may be possible for a more specific comparison between simulation results of different model parameters to be identified.

The method 100 further comprises a step 140 of generating output data 195, the output data 195 comprising indicators of all simulation results produced for all heart models.

Thus, output data 195 is generated that facilitates a comparison between different heart models. Each indicator may represent a different simulation result, so that an indicator is generated for each simulation result.

In some examples, the indicator for a simulation result comprises (e.g. some of) the data of the simulation result. For instance, where the simulation result comprises a predicted length for which re-entrant arrhythmia persists, the indicator may be a numeric measure containing the predicted length.

In other examples, the indicator for a simulation result comprises information derived by processing the simulation result. In particular, the indicator may provide a prediction of whether a cardiac event or arrhythmic event results from an electrical stimulation at the pacing location. For instance, where the simulation result comprises a predicted length for which re-entrant arrhythmia persists, the indicator may be a binary indicator that indicates whether or not the predicted length exceeds some predetermined threshold (e.g. 1 second), which may represent or indicate the occurrence of an arrhythmic event. In this way, an indicator may provide be a binary indicator of whether or not an arrhythmic event is predicted to result from an electrical stimulation at the particular pacing location.

Other examples of suitable indicators will be apparent to the skilled person.

In one example, the output data 195 is formed of one or more lists (e.g. a list for each heart model or each pacing location). Each list may contain indicators for all simulation results for that heart model or that pacing location. Each list may, for instance, be in the form of a vector.

In some examples, the output data 195 is formed of a matrix, in which each entry in the matrix represents a different simulation result. A matrix provides a simple and resource effective mechanism for storing, displaying and formatting indicators of simulation results. In particular, use of a matrix allows multi-dimensional formatting of simulation results, so that the simulation results for different models and pacing location can be arranged in an understandable fashion.

In some examples, entries of the matrix along a first dimension represent all simulation results of a same heart model.

In some examples, entries of the matrix along a second, different dimension represent simulation results of different heart models for a same pacing location. It is not essential that a value be provided for each position along the second dimension (e.g. for each model), as not all pacing locations need to be shared when simulating different models.

It will be appreciated that, in some examples, the matrix therefore represents the pacing locations on one axis/dimension and the different parameter settings on another axis/dimension. The entries in the matrix provide an indicator for each simulation result, e.g. a binary indicator that identifies whether arrhythmia has been observed in the simulations or not.

The output data 195 (matrix) provides information about the sensitivity of the simulation results with respect to the simulation parameters. If results for a particular pacing location are insensitive to parameter variations, then predictions likely correlate better with the actual condition of the subject and may reflect a low uncertainty in simulation outcome.

In particular, the risk of arrhythmia might be considered higher if arrhythmia is consistently predicted for one pacing location for different parameter settings. The overall risk of arrhythmia may be small if consistently no arrhythmia is predicted for all pacing conditions and all parameter variations.

The output data 195 may also be considered to provide an indication on the sensitivity of simulation with respect to certain parameters, and could be used to determine which (additional) parameters might be determined in addition to get a more accurate or precise simulation result. For example, parameter sets that are related to early-onset disease may warrant additional diagnostic procedures to exclude such disease, or early treatment if such disease is actually diagnosed.

The output data 195 results might, for instance, further indicate that additional data is required to better or more accurately personalize the heart model(s) to the specific subject, e.g. an indicator to obtain more accurate electrophysiological parameters, e.g. through using additional electrophysical mapping approaches.

The method 100 may be performed by a processing system. The step 110 may be performed by an input interface of the processing system (e.g. which communicates with a medical imaging system and/or a memory). The other steps 120 - 140 may be performed by a processing arrangement of the processing system.

Figure 2 illustrates an approach for constructing a heart model, which can be employed in step 120 of Figure 1. In particular, Figure 2 illustrates a diagram for generating a model of a heart from imaging data 205.

It will be appreciated that the illustrated approach is only provided for the purposes of understanding and by way of example only. Other approaches for generating one or more heart models will be apparent to the skilled person.

The imaging data 205 here comprises a magnetic resonance image of the heart of the subject. The imaging data 205 may, for instance, comprise a plurality of 2D images (e.g. taken at different angles and/or difference slices or positions of the heart) and/or a 3D image of the heart.

The imaging data 205 is segmented in a step 210, to identify different parts of the heart of the subject. The segmented imaging data comprises the original imaging data with the addition of one or more labels and/or annotations identifying parts of the heart represented in the imaging data (e.g. to identify the bounds of the heart and/or parts of the heart, such as the chambers and/or arteries of the heart). In particular, the segmented imaging data may identify the representation of the myocardium of the heart in the image data 205.

Approaches for automatically segmenting imaging data of a heart are well known to the skilled person, and may comprise processing the imaging data using a suitably trained machine-learning method (e.g. a neural network), one or more active contour mechanisms, one or more deformable model segmentation approaches and so on.

In a first pathway 220, the segmented imaging data is then processed in a step 221 to generate a structural model of the heart, e.g. a model of the chambers of the heart. Step 221 may, for instance, comprise a finite element model and/or a mesh-based model. Approaches for generating a structural model of the heart from segmented imaging data are well known. In some examples, the process performed in step 221 may be partially integrated into step 220 (e.g. if segmentation is performed by directly mapping a model to the imaging data).

Continuing the first pathway 220, the structural model is then processed in a step 222 that generates a fiber model of the heart (i.e. a model that represents a location, length and characteristics of fibers of the heart). Step 222 may use a rule-based mechanism for generating the fiber model, and such approaches are well known to the skilled person. The fiber model may therefore be a finite element model, where each fiber represents a finite element of the model.

In a second pathway 230, the segmented imaging data is processed in a step 231 to identify scar and/or gray zone tissue (and/or other infarcted tissue) in the segmented imaging data. This may be performed, for instance, by applying one or more thresholds or classifiers within the myocardium represented in the segmented imaging data.

Any identified scar and/or gray zone tissue is processed in a step 232 to generate an infarcted tissue model. Step 232 may, for instance, be performed using a shape-based interpolation method. The infarcted tissue model is a reconstruction of the infarcted (i.e. scar and/or gray zone tissue) tissue of the subject, and identifies a location and/or type of infarcted tissue.

The fiber model and infarcted tissue model can then be combined in a step 240 to generate the heart model. Step 240 may comprise assigning values to electrophysical properties of parts of the fiber model, to thereby produce the heart model (here: an electrophysical model of cardiac activity, which may comprise an ionic model of cardiac activity).

The infarcted tissue model may be used in the assignment of values for these electrophysical properties, e.g. by assigning different values for electrophysical properties assigned to each fiber of the fiber model depending upon the type of tissue represented by the fiber, as identified by the infarcted tissue model (e.g. whether the fiber is part of scar tissue, gray zone tissue or non-infarcted tissue).

Examples of suitable electrophysical properties include any human ventricular cell action potential dynamic property, e.g. peak sodium current, L-type calcium and potassium current, longitudinal and transversal conduction velocities and so on. By way of explanative example, in gray-zone regions, the transverse conductivity is reduced (compared to non-infarcted tissue), and scar tissue is considered to be electrically non-conductive.

As another example, in grey zone tissue, compared to non-infarcted tissue, there is a reduction in peak sodium current, in L-type calcium current and potassium currents. The values of these characteristics are irrelevant for scar tissue, as it is electrically non-conductive. The resulting gray zone action potential (e.g. during later simulation of the heart model) is characterized by a longer duration, decreased upstroke velocity and decreased peak amplitude compared with non-infarcted myocardium.

The values assigned to each electrophysical property may, at least in part, be based upon measured values (of the subject) and/or global/cohort values (e.g. values representing an average for a population or population cohort). Mechanisms for measuring or establishing values of the electrophysical property(ies) will be apparent to the skilled person, and may be defined by standard values. One approach is suggested by Sigg, Daniel C., et al., eds. Cardiac electrophysiology methods and models. Springer Science & Business Media, 2010. Other approaches may employ electrophysical mapping procedures that map the heart and infer values of electrophysical properties from the mapping procedure.

In this way, a heart model may effectively be an electrophysical model of cardiac activity. The heart model can then undergo simulation in order to assess characteristics of the modelled heart, and in particular, to predict characteristics of potential arrhythmia by simulating electrical stimulation at different pacing locations.

A more complete description of how to generate a heart model from imaging data, which could be adopted for the present disclosure, is found in H. Arevalo et al.; Arrhythmia risk stratification of patients after myocardial infarction using personalized heart models; Nature Communications 2016, 7, 11437.

From the foregoing description of how to generate a heart model, it will be apparent that there are a number of parameters or modifiable properties can define the heart model, and therefore affect simulation results produced using the heart model. There is therefore a range of possible parameters or modifiable properties that can be adjusted to define different heart models, and a range of potential values for each adjusted modifiable property.

For instance, it has been explained how infarcted tissue (e.g. scar tissue and/or gray zone tissue) can be identified by applying thresholds or classifiers to segmented image data. Variations of the thresholds or the use of slightly differently trained classifiers will lead to slightly different distributions of infarcted tissue, and therefore different heart models.

Thus, in some examples, to generate different heart models, different thresholds and/or classifiers may be used in the processing segmented image data to identified infarcted tissue.

It has also been explained how a fiber model can be generated from the structural model of the heart. This involves making use of one or more predetermined rules and/or parameters for the fibers, e.g. which define fiber length, width, structure, bendability and so on. Variations of these rules parameters will lead to different fiber models, and therefore different heart models. Thus, in some examples, to generate different heart models, different rules and/or (values for) parameters of fibers may be used in the generating of a fiber model.

It has also been explained how, in generating a model of the heart, different electrophysical properties can be assigned to fibers of the heart based on whether that fiber forms part of scar tissue, gray zone tissue or non-infarcted tissue. One or values for such parameters may be modified in order to define different heart models.

Of course, other approaches for generating heart models may be similarly adapted so that different heart models can be generated. There is an underlying understanding that each heart model has a different set of one or more modifiable properties. Thus, instead of a single reference set a of parameters for modeling a heart (or otherwise defining the model of the heart), a plurality of parameter sets a₁, a₂ ..., a_{N} can be used to generate a plurality of heart models.

Thus, the choice of one or more modifiable properties of the heart model may vary dependent upon the precise mechanism for generating and/or defining the heart model.

The values selected for the one or more modifiable parameters of the heart model may be defined so that the different models represent different situations or circumstances for the subject.

In some examples, different values may be chosen to represent natural population variation, so that at least some of the heart models represent natural variation within a population. It is known that (over a population) the true values for the one or more modifiable properties will vary, e.g. according to a bell curve or other distribution. Values for different heart models may be selected to represent variation within the population. For instance, values may be picked from different locations on their respective population distributions. As another example, a value may be based on a (population) mean value summed with differently weighted standard deviations or variances.

In some examples, different values may be chosen to represent natural variation within a particular population cohort or between different population cohorts. In one example, natural variation within a population cohort to which the subject belongs is used to control the values that are selected. This adopts a similar approach to the entire population, previously described, but for a specific population cohort.

In yet other examples, values may be chosen based on measurement uncertainty for a particular modifiable property, e.g. may be based on error margins (e.g. within a range of ± 1% or ±5%).

In some examples, different values may be chosen to represent the effect of different drugs and/or treatment options. For instance, it may be known that an ablation will increase the presence of grey zone or scar tissue in the heart, which information can be used to define different heart models. As another example, it may be known that certain drugs will affect particular properties of the heart (e.g. lithium or lithium chloride may reduce a peak sodium current). Thus, different heart models may be generated to represent the effects of different drugs and/or treatment options on the heart.

Of course, a combination of these approaches may be adopted, so that different values are chosen to represent natural population (cohort) variation, measurement uncertainty and/or the effect of different drugs and/or treatment options.

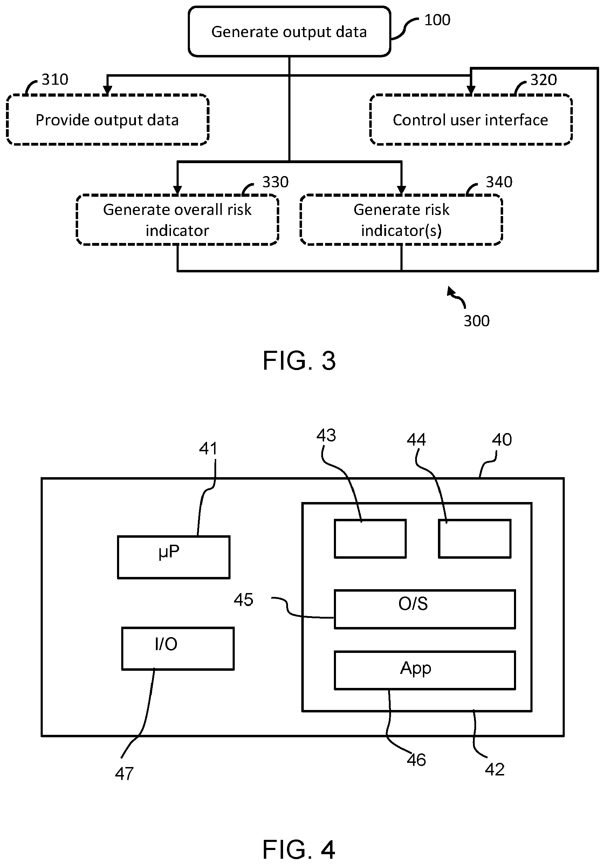

Figure 3 illustrates a method 300 according to an embodiment. The method comprises a process 100 of generating output data, which may adopt any previously described approach.

The method 300 may comprise a step 310 of outputting the output data to a user interface or (further) processor. The output data contains information that would be advantageous for viewing by a clinician or user (e.g. at a user interface) or for further processing, e.g. to control one or more future actions.

The method 300 may comprise a step 320 of controlling a user interface to provide a visual representation of the output data. The exact nature of the visual representation may depend upon the content of the output data. For instance, where the output data is in the form of a matrix, a visual representation of the matrix may be provided at the user interface.

As another example, where the output data comprises one or more lists (e.g. one or more vectors), a visual representation of each list may be provided at the user interface. In some examples, a user may be able to interact with an identifier for each list (e.g. for each heart model or each pacing location) so that display of a visual representation of the list is responsive to a user input. If each list represents a pacing location, the identifier for the list may be displayed to overlay a visual representation of the heart, e.g. at an appropriate position representing the pacing location with respect to the heart.

The method 300 may be performed by a processing system. The step 310 may be performed by an output interface of the processing system. Step 320 may be performed by a processing arrangement of the processing system (which may control the user interface via an output interface of the processing system).

The output data may be further processed to generate one or more risk indicators.

The method 300 may comprise, for instance, a step 330 of processing the output data, e.g. the indicators of all simulation results, in order to generate an overall risk indicator.

By way of explanation, in the event that simulation results (or indicators) for a particular pacing location are insensitive to using different models, then predictions likely correlate better with the actual patient condition and may reflect a low uncertainty in simulation outcome. Thus, the risk of arrhythmia may be considered higher if arrhythmia is consistently predicted for one pacing location using different heart models. Similarly, the arrhythmia risk may be particularly small, if consistently no arrhythmia is predicted for all pacing conditions across different heart models.

Thus, indicators of simulation results can be processed in order to assess an overall risk of arrhythmia to the heart of the subject. As the heart models are all built from the same imaging data (of a particular subject), the proposed approach provides a more robust solution to assessing risk to the subject's heart.

By way of example, if the output data comprises (for each simulation result) a binary indicator of whether or not arrhythmia is detected for a particular pacing location, step 330 may comprise determining whether (for any of the pacing locations) more than a predetermined fraction or percentage of the binary indicators indicate that arrhythmia is predicted. In response to, for any of the pacing locations, more than a predetermined fraction/percentage of the binary indicators indicate that arrhythmia is predicted, the overall risk indicator may indicate that arrhythmia of the subject is predicted. Otherwise, the overall risk indicator may indicate that arrhythmia of the subject is not predicted.

Step 330 may comprise, for example, processing the (indicators of the) output data using a machine-learning method to generate the overall risk indicator.

In an example, the method may comprise a step 340 of processing the output data to generate, for each pacing location, a risk indicator representing a risk of arrhythmic activity for each pacing location.

By way of example, if the output data comprises (for each simulation result) a binary indicator of whether or not arrhythmia is detected for a particular pacing location, step 340 may comprise determining, for each pacing location, whether or not more than a predetermined fraction or percentage of the binary indicators indicate that arrhythmia is predicted. This may act as the risk indicator for that pacing location.

In other examples, step 340 may comprise, for example, processing, for each pacing location, the indicators of the output data associated with that pacing location using a machine-learning method to generate the risk indicator for that pacing location.

If step 330 and/or step 340 is performed, step 320 may be adapted to provide a visual representation of the indicator(s) generated in either of these steps.

Purely by way of example, a visual representation of the heart may be provided at the user interface. A position of each pacing location may be indicated on the visual pacing location. A visual representation of a risk indicator (e.g. using a red/green color indicator or gradient indicator) for each pacing location (generated in step 340) may be provided at the position of each pacing location and/or alongside the position of each pacing location. Interaction with the visual representation of the pacing location and/or the pacing location may cause indicators of all simulation results for that pacing location to be generated. This embodiment may make use of lists of (indicators or) simulation results that may be produced for each pacing location. This embodiment provides a clinician with an approach to assessing the risk at each pacing location and/or the sensitivity of the heart models to change at particular pacing locations. This approach facilitates improved identification of potential areas of concern for the cardiac structure of the subject.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system that employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

By way of further example, Figure 4 illustrates an example of a processing system 40 within which one or more parts of an embodiment may be employed. Various operations discussed above may utilize the capabilities of the processing system 40. For example, one or more parts of a processing system for processing medical imaging data to produce output data may be incorporated in any element, module, application, and/or component discussed herein. In this regard, it is to be understood that system functional blocks can run on a single computer or may be distributed over several computers and locations (e.g. connected via internet).

The processing system 40 includes, but is not limited to, PCs, workstations, laptops, PDAs, palm devices, servers, storages, and the like. The processing system may be part of a dielectric imaging system and/or electrophysiological or cardiac mapping system as for example known in the art and commercially available in the form of for example the Carto^{™} mapping system, the EnSite Precision^{™} cardiac mapping system, the Rythmia^{™} mapping system, the AcQMap^{™} system or any other such system. The system may be part of a console in such dielectric imaging systems and or in a separate workstation of such a system. Generally, in terms of hardware architecture, the processing system 40 may include one or more processing arrangements 41, memory 42, and one or more I/O devices 47 that are communicatively coupled via a local interface (not shown). The local interface can be, for example but not limited to, one or more buses or other wired or wireless connections, as is known in the art. The input interface and/or output interface are configured to be able to communicate with the processor(s). For example data and commands may be communicated. There may be other inputs and outputs too. The local interface may have additional elements, such as controllers, buffers (caches), drivers, repeaters, and receivers, to enable communications. Further, the local interface may include address, control, and/or data connections to enable appropriate communications among the aforementioned components.

The processing arrangement 41 is a hardware device for executing software that can be stored in the memory 42. The processing arrangement 41 can be virtually any custom made or commercially available processing arrangement, a central processing unit (CPU), a digital signal processing arrangement (DSP), or an auxiliary processing arrangement among several processing arrangements associated with the processing system 40, and the processing arrangement 41 may be a semiconductor based microprocessing arrangement (in the form of a microchip) or a microprocessing arrangement.

The memory 42 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 42 may incorporate electronic, magnetic, optical, and/or other types of storage media. Note that the memory 42 can have a distributed architecture, where various components are situated remote from one another, but can be accessed by the processing arrangement 41.

The software in the memory 42 may include one or more separate programs, each of which comprises an ordered listing of executable instructions for implementing logical functions. The software in the memory 42 includes a suitable operating system (O/S) 45, compiler 44, source code 43, and one or more applications 46 in accordance with exemplary embodiments. As illustrated, the application 46 comprises numerous functional components for implementing the features and operations of the exemplary embodiments. The application 46 of the processing system 40 may represent various applications, computational units, logic, functional units, processes, operations, virtual entities, and/or modules in accordance with exemplary embodiments, but the application 46 is not meant to be a limitation.

The operating system 45 controls the execution of other computer programs, and provides scheduling, input-output control, file and data management, memory management, and communication control and related services. It is contemplated by the inventors that the application 46 for implementing exemplary embodiments may be applicable on all commercially available operating systems.

Application 46 may be a source program, executable program (object code), script, or any other entity comprising a set of instructions to be performed. When a source program, then the program is usually translated via a compiler (such as the compiler 44), assembler, interpreter, or the like, which may or may not be included within the memory 42, so as to operate properly in connection with the O/S 45. Furthermore, the application 46 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 47 may include input devices such as, for example but not limited to, a mouse, keyboard, scanner, microphone, camera, etc. Furthermore, the I/O devices 47 may also include output devices, for example but not limited to a printer, display, etc. Finally, the I/O devices 47 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 47 also include components for communicating over various networks, such as the Internet or intranet.

In particular, the I/O devices 47 may comprise an input interface, e.g. configured to communicate with a medical imaging system and/or memory that obtains medical imaging data for processing by the processing arrangement 41 of the processing system 40.

In some examples, the I/O devices 47 may comprise an output interface configured to output the output data, e.g. to a user interface or the like. In some examples, the user interface is integrated as an aspect of the I/O devices 47.

If the processing system 40 is a PC, workstation, intelligent device or the like, the software in the memory 42 may further include a basic input output system (BIOS) (omitted for simplicity). The BIOS is a set of essential software routines that initialize and test hardware at startup, start the O/S 45, and support the transfer of data among the hardware devices. The BIOS is stored in some type of read-only-memory, such as ROM, PROM, EPROM, EEPROM or the like, so that the BIOS can be executed when the processing system 40 is activated.

When the processing system 40 is in operation, the processing arrangement 41 is configured to execute software stored within the memory 42, to communicate data to and from the memory 42, and to generally control operations of the processing system 40 pursuant to the software. The application 46 and the O/S 45 are read, in whole or in part, by the processing arrangement 41, perhaps buffered within the processing arrangement 41, and then executed.

When the application 46 is implemented in software, it should be noted that the application 46 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 46 can be embodied in any computer-readable medium for use by or in connection with an instruction execution system, apparatus, or device, such as a computer-based system, processing arrangement-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

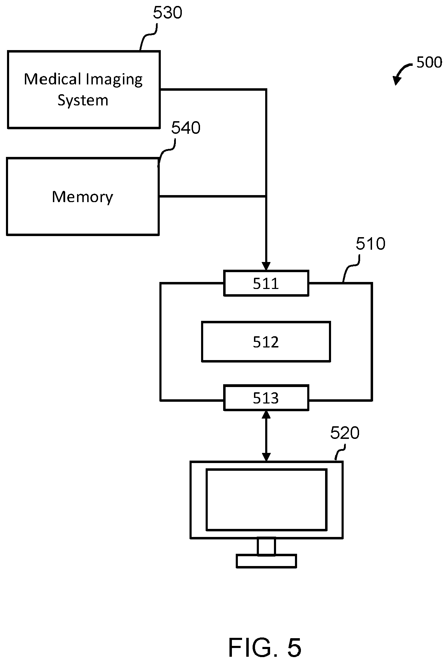

Figure 5 illustrates a system 500 according to an embodiment of the invention. The system comprises a processing system 510.

The processing system 510 comprises an input interface 511 configured to obtain medical image data of the heart of a subject.

The processing system 510 also comprises a processing arrangement 512 configured to: construct a plurality of different heart model of the heart of the subject by processing the medical image data, wherein each heart model is defined by a different set of one or more values for one or more modifiable properties of the heart model; for each heart model, perform two or more simulations of electrophysiologic activity, wherein each simulation is carried out using a different pacing location with respect to the heart model, to produce a respective two or more simulation results; and generate output data, the output data comprising indicators of all simulation results produced for all heart model.

The processing system 510 may optionally further comprise an output interface 513 configured to output the output data.

The processing system may be embodied as the processing system described with reference to Figure 4. However, this is not essential and other configurations for a processing system will be apparent to the skilled person.

The system 500 may further comprise a user interface 520, configured to receive (e.g. via the output interface 513) a control signal from the processing system. The control signal controls the user interface to provide a visual representation of the output data. Thus, the processing arrangement 511 may be configured to control the user interface (e.g. via the output interface) to provide a visual representation of the output data.

The system 500 may further comprise a medical imaging system 530. The medical imaging system is configured to generate medical image data of the heart of a subject. The medical image data is suitable for processing to generate a plurality of heart models of the heart of the subject. Suitable examples of medical imaging systems include MRI scanners, ultrasound scanner, CT or X-ray scanners, PET scanners and so on.

The system may comprise a memory or storage module 540. The memory or storage module may provide the medical imaging data to the input interface 511 of the processing system 510. The medical imaging data may be originally generated by the medical imaging system 530 or another medical imaging system (not shown).

Thus, the input interface may be configured to receive the medical imaging data from the medical imaging system 530 or the memory 540. Both the medical imaging system and the memory are optional modules of the system 500.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (100, 300) of modelling the heart of a subject, the computer-implemented method comprising:
obtaining (110) medical image data (105) of the heart of a subject;
constructing (120) a plurality of different heart models of the heart of the subject by processing the medical image data, wherein each heart model is defined by a different set of one or more values for one or more modifiable properties of the heart model;
for each heart model, performing (130) two or more simulations of electrophysiologic activity, wherein each simulation is carried out using a different pacing location with respect to the heart model, to produce a respective two or more simulation results for the heart model; and
generating (140) output data (195), the output data comprising indicators of all simulation results produced for all heart models.

2. The computer-implemented method (300) of claim 1, further comprising a step of processing (340) the output data to generate, for each pacing location, a risk indicator representing a risk of arrhythmic activity for each pacing location.

3. The computer-implemented method (300) of claim 1 or 2, further comprising a step of processing (330) the output data to generate an overall risk indicator representing a risk of arrhythmic activity for the subject.

4. The computer-implemented method of any of claims 1 to 3, wherein the differences between different sets of one or more values for one or more modifiable properties represent variations between different individuals, variations between different population cohorts, measurement uncertainty and/or the influence of different drugs and/or treatments on the heart.

5. The computer-implemented method of any of claims 1 to 4, wherein a simulation result is a measure of arrhythmic activity.

6. The computer-implemented method of claim 5, wherein the measure of arrhythmic activity is a measure of a predicted length of reentrant arrhythmia.

7. The computer-implemented method of claim 5 or 6, wherein each indicator comprises a classification of whether or not arrhythmia is predicted, the classification being produced by processing the measure of arrhythmic activity.

8. The computer-implemented method of any of claims 1 to 7, wherein each heart model is an electrophysical model of cardiac activity.

9. The computer-implemented method of claim 8, wherein the one or more modifiable properties comprise one or more electrophysiological properties of tissue represented by each heart model.

10. The computer-implemented method of any of claims 1 to 9, wherein one or more modifiable properties of the heart comprise an amount and/or location of scar tissue, gray zone tissue and/or non-infarcted tissue of the heart.

11. The computer-implemented method of any of claims 1 to 10, further comprising a step (310) of outputting the output data to a user interface (520) or processor.

12. The computer-implemented method of any of claims 1 to 11, further comprising a step (320) of controlling a user interface (520) to provide a visual representation of the output data.

13. A computer program product comprising computer program code means which, when executed on a computing device having a processing system, cause the processing system to perform all of the steps of the computer-implemented method according to any of claims 1 to 12.

14. A processing system (40, 510) configured to model the heart of a subject, the processing system comprising:
an input interface (511) configured to obtain (110) medical image data of the heart of a subject;
a processing arrangement (512) configured to:
construct (120) a plurality of different heart model of the heart of the subject by processing the medical image data, wherein each heart model is defined by a different set of one or more values for one or more modifiable properties of the heart model;
for each heart model, perform (130) two or more simulations of electrophysiologic activity, wherein each simulation is carried out using a different pacing location with respect to the heart model, to produce a respective two or more simulation results; and
generate (140) output data, the output data comprising indicators of all simulation results produced for all heart model,
wherein the processing system optionally further comprises an output interface (513) configured to output (310) the output data.

15. A system (500) comprising:
the processing system (510) of claim 14; and
a user interface (520), wherein the processing arrangement is configured to control the user interface to provide a visual representation of the output data.
